(19) Europäisches Patentamt
European Patent Office
Office européen des brevets

(11) Veröffentlichungsnummer : **0 043 950**
**B1**

# (12) EUROPÄISCHE PATENTSCHRIFT

(45) Veröffentlichungstag der Patentschrift : **05.09.84**

(21) Anmeldenummer : **81104877.6**

(22) Anmeldetag : **24.06.81**

(51) Int. Cl.³ : **C 07 C 61/15**, C 07 C 69/74, C 07 C 51/377, C 07 C 67/317

## (54) 1-Methyl-2-chlorcyclopropancarbonsäure und deren Ester sowie Verfahren zu deren Herstellung.

(30) Priorität : **10.07.80 DE 3026093**

(43) Veröffentlichungstag der Anmeldung : **20.01.82 Patentblatt 82/03**

(45) Bekanntmachung des Hinweises auf die Patenterteilung : **05.09.84 Patentblatt 84/36**

(84) Benannte Vertragsstaaten : **BE CH FR GB IT LI NL**

(56) Entgegenhaltungen :
**FR-A- 2 121 780**
**FR-A- 2 378 740**
**ACTA CHEMICA SCANDINAVICA, Band 32, Nr. 9, September 1978, Seiten 632-638 L.KR. SYDNES et al.: "Chemistry of gem-dihalocyclopropanes. XV. Reactions of gem. dihalocyclopropanes with sodium bis (2-methoxyethoxy)-aluminium hydride, lithium aluminium hydride, and sodium borohydride"**
**Die Akte enthält technische Angaben, die nach dem Eingang der Anmeldung eingereicht wurden und die nicht in dieser Patentschrift enthalten sind.**

(73) Patentinhaber : **Degussa Aktiengesellschaft**
**Weissfrauenstrasse 9**
**D-6000 Frankfurt am Main 1 (DE)**

(72) Erfinder : **Schwarze, Werner, Dr.**
**Leerbachstrasse 117**
**D-6000 Frankfurt (Main) (DE)**
Erfinder : **Kleemann, Axel, Dr.**
**Greifenhagenstrasse 9**
**D-6450 Hanau 9 (DE)**
Erfinder : **Leuchtenberger, Wolfgang, Dr.**
**Geschwister-Scholl-Strasse 1**
**D-6454 Bruchköbel (DE)**

**EP 0 043 950 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Cyclopropancarbonsäuren sind wichtige Zwischenprodukte für die Herstellung von Pesticiden und Pharmazeutica. Ihre Ester können zur Bekämpfung von Milben verwendet werden (DE-AS 24 17 372). 1-Methylcyclopropylcarboxananilido-Derivate können als selektive Herbicide in Sojakulturen verwendet werden (US-PS 4 168 153).

Das 4-Amino-6-(1-methylcyclopropyl)-3-methylthio-1.2.4-triazin-5-on stellt ein hochwirksames Herbicid dar (BE-PS 869 138). Für seine Synthese wird die 1-Methyl-Cyclopropancarbonsäure benötigt.

Andere Cyclopropancarbonsäuren, z. B. die 2-(2.2-Dihalovinyl)-3,3-dialkylcyclopropancarbonsäure und ihre Ester sind wichtige Schlüsselsubstanzen für die Synthese einer Gruppe von Verbindungen, die unter der Bezeichnung « synthetische Pyrethroide » bekannt sind und die eine bemerkenswerte insecticide und acaricide Wirkung besitzen.

Gegenstand dieser Erfindung ist eine neue Cyclopropancarbonsäure, nämlich die 1-Methyl-2-chlorcyclopropancarbonsäure, sowie deren Ester. Diese Verbindungen entsprechen der allgemeinen Formel

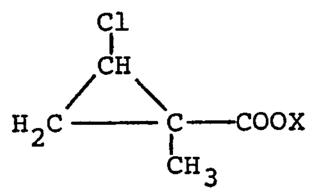

(I)

in der X für ein Wasserstoffatom oder für eine verzweigte oder unverzweigte Alkylgruppe mit 1-6 C-Atomen steht.

Diese Verbindungen zeichnen sich dadurch aus, daß sie im Gegensatz zu den unsubstituierten Cyclopropancarbonsäuren, bzw. -estern, einen stabilen Cyclopropanring besitzen, der z. B. durch Säuren nicht spaltbar ist. Die neuen Verbindungen können erfindungsgemäß aus Verbindungen der allgemeinen Formel

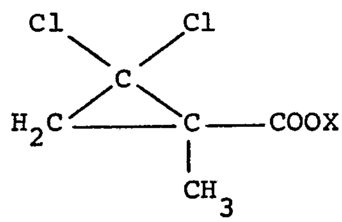

(II)

in der X ein Wasserstoffatom oder eine verzweigte oder unverzweigte Alkylgruppe mit 1-6 C-Atomen bedeutet, durch selektive Hydrierung in Gegenwart eines Edelmetallkatalysators und in Gegenwart eines HCl-Acceptors und gegebenenfalls in Abwesenheit eines Lösungsmittels hergestellt werden. Geht man hierbei von der freien Säure aus, so ist es erforderlich, diese zuvor in die Salzform zu überführen.

Aus der FR-PS 2 378 740 ist es bekannt 2-Chlor-3-alkylcyclopropancarbonsäuren aus den entsprechenden 2-Dichlorverbindungen mittels einer hydrierenden Dechlorierung herzustellen. Diese Reaktion wird jedoch unter Verwendung von Zinkpulver oder einer Trialkyl-zinn-wasserstoff-Verbindung durchgeführt. Offenbar können bei den in dieser Patentschrift beschriebenen Ausgangsverbindungen bei Verwendung eines Edelmetallkatalysators keine definierten Reaktionsabläufe erzielt werden. Demgegenüber war es überraschend, daß nach dem beanspruchten Verfahren sowohl die freie 1-Methyl-2,2-dichlorcyclopropancarbonsäure als auch ihre Ester definiert zur Monochlorsäure hydrierend dechloriert werden können. Es war dabei insbesondere überraschend, daß die Ester — beispielsweise in Gegenwart von Ammoniak — bei erhöhter Temperatur hydrierend dechloriert und nicht verseift bzw. aminiert werden.

Die 1-Methyl-2,2-dichlorcyclopropancarbonsäureester können in sehr hohen Ausbeuten aus Methacrylsäureester und Chloroform in Gegenwart hochprozentigen Alkalis, z. B. NaOH oder KOH, und in Gegenwart eines Phasentransfer-Katalysators, z. B. Triäthylbenzylammoniumchlorid, hergestellt werden. (E.V. Dehmlow, Liebigs Ann. 758, p. 148-152 (1972)). Die so erhaltenen Ester können leicht ohne Zerstörung des Cyclopropanringes mit Alkali verseift werden.

Die erfindungsgemäße selektive hydrierende Dechlorierung kann in Wasser, Wasser-Lösungsmittel-Gemischen, z. B. in Methanol, Äthanol, n-Propanol, i-Propanol, n-Butanol, i-Butanol, sec-Butanol, t-Butanol, Dioxan und Tetrahydrofuran durchgeführt werden. Es ist aber auch möglich, wasserfrei zu arbeiten, wenn man von den flüssigen Estern ausgeht und als HCl-Acceptor wasserfreien Ammoniak einsetzt. Vorzugsweise arbeitet man in Wasser sowie in Methanol oder Äthanol, bzw. in Gemischen aus diesen.

Die im Rahmen dieser Erfindung zu verwendenden Hydrierkatalysatoren sind an sich bekannt. Ihre Herstellung ist beispielsweise beschrieben in Adams et al, Am. Soc. 45, p 2175-78 (1923). Die Technik der Herstellung von Fällungs- und Imprägnier- sowie Tränkkatalysatoren ist eingehend in Ullmann, 4. Auflage, Band 13 ab p 558 beschrieben worden.

Es kommen hierfür insbesondere die Edelmetalle Platin und Palladium in Betracht. Bevorzugt verwendet man jedoch Palladium-Katalysatoren auf Trägermaterialien, insbesondere auf Aktiv-Kohle. Als weitere Trägermaterialien können z. B. Kieselgele, Kieselgur, Aluminiumoxyde, Zeolithe, Bimsstein sowie verschiedene Silicate verwendet werden.

Als HCl-Acceptoren können insbesondere wässrige Lösungen von Natriumhydroxid und Kaliumhydroxid oder Ammoniak verwendet werden. Weiter kommen in Frage : Lithiumhydroxid, Natrium-, Kalium- und Lithiumhydrogencarbonat und Carbonate, Hydroxide und Carbonate von Erdalkalien, organische tertiäre Amine, z. B. Trimethylamin, Triäthylamin, Ammoniak, Ammonhydrogencarbonat und -carbonat. Beim wasserfreien Arbeiten kann man Alkoholate insbesondere Natriummethylat oder -äthylat oder wasserfreien Ammoniak verwenden. Bevorzugt setzt man Natriummethylat ein.

Geht man bei der Durchführung des erfindungsgemäßen Verfahrens von der freien 1-Chlor-2,2-dichlorcyclopropancarbonsäure aus, so ist es erforderlich diese zuerst in die Salzform zu überführen, was beispielsweise mit Alkali in an sich bekannter Weise erfolgen kann. In diesem Falle muß man selbstverständlich für die dehydrierende Dechlorierung ein weiteres Mol des HCl-Acceptors pro Mol Säure aufwenden. Man kann tertiäre organische Amine sowohl in normalen Lösungsmitteln als auch in Wasser als HCl-Acceptoren verwenden. Zum Beispiel kann man die freie Ausgangssäure z. B. in Wasser mit Triäthylamin in die Salzform überführen und als HCl-Acceptor nochmals Triäthylamin anwenden. Man kann aber auch Mischungen von organischen t-Aminen und anorganischen Alkalien verwenden. Es ist zweckmäßig den HCl-Acceptor in einem kleinen Überschuß von beispielsweise 10 % einzusetzen.

Geht man bei der Durchführung des erfindungsgemäßen Verfahrens von Verbindungen der allgemeinen Formel (II) aus, in der X für eine Alkylgruppe steht, setzt man also die Ester als Ausgangsstoffe ein, so ist es zweckmäßig als HCl-Acceptor Ammoniak zu verwenden. Dieses kann sowohl als wässrige Lösung als auch wasserfrei eingesetzt werden. In beiden Fällen erfolgt während der Hydrierung keine Verseifung und auch keine Überführung in das Amid.

Die hydrierende Dechlorierung kann bei Drucken zwischen 10 und 250 bar, vorzugsweise bei Drucken von 50 bis 150 bar durchgeführt werden.

Die Hydrierung kann ferner bei Temperaturen zwischen 20 °C und 120 °C, vorzugsweise zwischen 50 °C und 80 °C durchgeführt werden.

Die neuen Verbindungen gemäß Formel (I) sind farblose destillierbare Öle. Sie sind in jedem Fall cis-trans-Isomere.

In den Formeln (I) und (II) kann X z. B. für den Methyl-, Äthyl-, n-Propyl-, i-Propyl-, n-Butyl-, sec-Butyl-, i-Butyl-, t-Butyl-, n-Pentyl-, i-Pentyl-, n-Hexyl-, 1-Methylpentyl und 2-Methylpentylrest stehen.

Beispiel 1

934 g 1-Methyl-2,2-dichlorocyclopropancarbonsäuremethyl-ester (98 %-ig entsprechend 5 Mol) tropft man innerhalb einer Stunde in eine Lösung von 225 g KOH in 1 800 ml $CH_3OH$. Die Temperatur soll dabei 30 °C nicht übersteigen. Nach 4 Stunden ist die Verseifung beendet, dann wird das Methanol abdestilliert und entsprechend mit Wasser ergänzt (1 800 ml). Zu dieser farblosen Lösung gibt man noch einmal unter Kühlung 225 g festes KOH zu, fügt 50 g Pd-A-Kohle (5 %) hinzu, überführt alles in einen 5 Liter-Autoklaven, verschließt diesen und drückt 50 bar Wasserstoff auf. Reaktionstemperatur : 50 °C. Die Hydrierung ist in 6 Stunden beendet. Man entleert den Autoklaven, filtriert vom Katalysator ab und kühlt die Lösung auf 10 °C ab. Dann fügt man 1,5 Liter Methylenchlorid hinzu. Nun wird unter guter Kühlung zwischen 5 °C und 15 °C konzentrierte (38 %-ige) HCl zugegeben und auf $PH_1$ eingestellt. Die $CH_2Cl_2$-Schicht wird abgetrennt, getrocknet und an einer Kolonne destilliert (1 m, Maschendraht).

Bei $Kp_{14}$ 114-115 °C destilliert 1-Methyl-2-chorcyclopropancarbonsäure.

Analyse : $C_5H_7ClO_2$ (Mol-Gew. 134,5)

ber. C 44,6 H 5,2 Cl 26,2

gef. C 44,8 H 5,3 Cl 25,9

Menge : 525,2 g, entsprechend 78,1% d. Th. NMR- und GC-Analyse zeigen, daß die Säure ein cis-trans- Gemisch ist. (89 : 11).

Beispiel 2

732 g 1-Methyl-2,2-dichlorocyclopropancarbonsäuremethylester ( = 4 Mol) mischt man mit 400 ml Methanol. Die Mischung gibt man in einen 5-Liter-Autoklaven, gibt 18 g Pd-A-Kohle (5 %) hinzu und verschließt. Nun drückt man 136 g $NH_3$ (= 200 ml $NH_3$) auf, anschließend Wasserstoff bis zu einem Gesamtdruck von 100 bar. Bei 60 °C springt die Hydrierung an, sie ist in 5 Stunden beendet. Der Autoklav wird geöffnet, die Lösung filtriert und zuerst im Vakuum eingeengt und vom $NH_4Cl$ abgesaugt. Der Rest wird im Vakuum an einer 1 m-Raschig-Kolonne rektifiziert.

Bei $Kp_{12}$ 57-61 °C destilliert der 1-Methyl-2-chlorcyclopropancarbonsäuremethylester.
Analyse : $C_6H_9ClO_2$ (Mol-Gew. 148,5)
ber. C 48,5 H 6,1 Cl 23,8
gef. C 48,5 H 6,0 Cl 23,6
Menge : 482,3 g, entsprechend 81,2 % d. Th.
Der Ester ist ein cis- trans- Gemisch (NMR und GC-Analyse).

Beispiel 3

450 g 1-methyl-2,2-dichlorcyclopropancarbonsäure-n-butylester (2 Mol) gibt man in einen 2 1-Autoklaven, fügt 400 ml konz. wässrigen Ammoniak (ca. 25 %, ca. 4 Mol) und 10 g Pd-A-Kohle (10 %) hinzu, verschließt, drückt 100 bar $H_2$ auf und erwärmt. Bei 50 °C setzt die Hydrierung ein, sie ist in 4 Stunden beendet. Man filtriert, trennt die wässrige Lösung und destilliert 1-Methyl-2-chlorcyclopropancarbonsäure-n-butylester.
$Kp_{12}$, 99°-100 °C.
Analyse : $C_9H_5ClO_2$ (Mol-Gew. 190,5)
ber. C 56,7 H 7,9 Cl 18,6
gef. C 56,6 H 7,8 Cl 18,4
Menge : 305,6 g, entsprechend 80,2 % d. Th.

Beispiel 4

Man löst 338 g kristalline 1-Methyl-2,2-dichlorcyclopropancarbonsäure in 166 g NaOH + 1 l Wasser, gibt alles in einen 2 l-Autoklaven, fügt 10 g pt-A-Kohle (5 %-ig) hinzu, verschließt, drückt 100 bar $H_2$ auf und hydriert bei 50-60 °C.

Die Aufarbeitung erfolgt wie in den vorgängigen Beispielen beschrieben und liefert 211,2 g 1-Methyl-2-chlorcyclopropancarbonsäure, entsprechend 78,5 % d. Th.

Beispiel 5

Man löst 382 g 1-Methyl-2,2-dichlorcyclopropansaures Natrium (= 2 Mol) in 1 l Wasser, fügt 88 g NaOH und 5 g $PtO_2$ hinzu und gibt alles in einen 4 l-Autoklaven. Man verschließt und drückt 100 bar $H_2$ auf. Die Hydrierung erfolgt zwischen 60 °C und 75 °C. Sie ist in 6 Stunden beendet.

Die übliche Aufarbeitung liefert 212,8 g 1-Methyl-2-chlorcyclopropancarbonsäure vom $Kp_{14}$ 113-116 °C.
Ausbeute : 79,1 % d. Th.

Beispiel 6

Man gibt 253 g 1-Methyl-2,2-dichlorcyclopropancarbonsäure-n-hexylester in einen 2 l-Autoklaven, fügt 200 ml Methanol und 10 g Palladium auf Aktiv-Kohle (10 %-ig) hinzu, verschließt und drückt 100 g Ammoniak auf.

Wasserstoff wird aufgepreßt (100 bar) und auf 80°-90 °C erwärmt. Die Hydrierung ist in 6 Stufen beendet. Man gibt den Inhalt des Autoklaven in 2 l Wasser und schüttelt mit Methylenchlorid aus. Nach Abtreiben des Lösungsmittels wird im Vakuum destilliert, $Kp_{17}$ 132,5-133,5 °C.
Analyse : $C_{11}H_{19}O_2Cl$ (Mol-Gew. 218,5)
ber. C 60,4 H 8,7 Cl 16,2
gef. C 60,3 H 8,5 Cl 16,0
Ausbeute an 1-Methyl-2-chlorcyclopropancarbonsäure-n-hexylester : 180,5 g, entsprechend 82,6 % d. Th.

**Ansprüche**

1. 1-Methyl-2-chlorcyclopropanverbindungen der Formel (I)

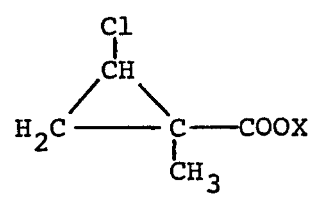

(I)

in der X ein Wasserstoffatom oder eine unverzweigte oder verzweigte Alkylgruppe mit 1-6 C-Atomen bedeutet.

2. Verfahren zur Herstellung von Verbindungen gemäß Anspruch 1, dadurch gekennzeichnet, daß man 1-Methyl-2,2-dichlorcyclopropanverbindungen der Formel (II)

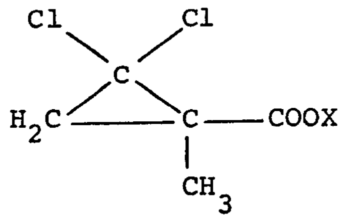

(II)

in der X ein Wasserstoffatom oder eine Alkylgruppe mit 1-6 C-Atomen bedeutet, in Gegenwart eines an sich bekannten Edelmetallkatalysators und in Gegenwart eines HCl-Acceptors selektiv hydrierend dechloriert, wobei zuvor, falls X ein Wasserstoffatom bedeutet, die freie Säure in die Salzform überführt wird.

3. Verfahren nach Anspruch 2, dadurch gekennzeichnet, daß man in Abwesenheit eines Lösungsmittels selektiv hydrierend dechloriert.

4. Verfahren nach Anspruch 2, dadurch gekennzeichnet, daß man die Dechlorierung in Gegenwart von Wasser durchführt.

5. Verfahren nach den Ansprüchen 2 bis 4, dadurch gekennzeichnet, daß man als HCl-acceptoren wässrige Alkalihydroxid- oder Ammoniak-Lösungen oder wasserfreies Ammoniak oder Alkoholate verwendet.

6. Verfahren nach den Ansprüchen 2 bis 5, dadurch gekennzeichnet, daß man bei Drucken zwischen 10 und 250 bar und bei Temperaturen zwischen 20 °C und 120 °C arbeitet.

7. Verfahren nach den Ansprüchen 2 bis 6, dadurch gekennzeichnet, daß man als Katalysatoren Palladium und Platin verwendet.

8. Verfahren nach den Ansprüchen 2 bis 7, dadurch gekennzeichnet, daß man als Katalysator Palladium auf Aktiv-Kohle verwendet.

**Claims**

1. 1-methyl-2-chlorocyclopropane compounds corresponding to formula (I)

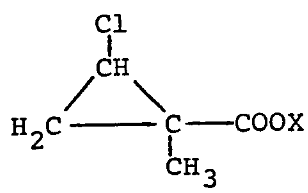

(I)

in which X represents a hydrogen atom or an unbranched or branched alkyl group having from 1 to 6 carbon atoms.

2. A process for the production of compounds according to claim 1, characterised in that 1-methyl-2,2-dichlorocyclopropane compounds corresponding to formula (II)

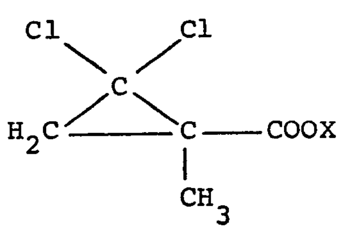

(II)

in which X represents a hydrogen atom or an alkyl group having from 1 to 6 carbon atoms, are selectively dechlorinated by hydrogenation in the presence of a known precious metal catalyst and in the presence of an HCl-acceptor, the free acid previously having been converted into the form of salt, if X represents a hydrogen atom.

3. A process according to claim 2, characterised in that the selective dechlorination by hydrogenation is carried out in the absence of a solvent.

4. A process according to claim 2, characterised in that dechlorination is carried out in the presence of water.

5. A process according to claims 2 to 4, characterised in that aqueous alkali metal hydroxide solutions or ammonia solutions or anhydrous ammonia or alcoholates are used as HCl-acceptors.

6. A process according to claims 2 to 5, characterised in that the process is carried out under a pressure of from 10 to 250 bars and at a temperature of from 20 to 120 °C.

7. A process according to claims 2 to 6, characterised in that palladium and platinum are used as catalysts.

8. A process according to claims 2 to 7, characterised in that palladium on active carbon is used as catalyst.

**Revendications**

1. Dérivés du 1-méthyl-2-chlorocyclopropane de formule (I)

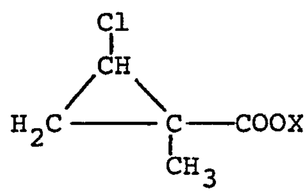

(I)

où X représente un atome d'hydrogène ou un groupe alcoyle ramifié ou non, comportant 1 à 6 atomes de carbone.

2. Procédé d'obtention des composés selon la revendication 1, caractérisé en ce que l'on soumet le dérivé du 1-méthyl-2,2-dichlorocyclopropane de formule (II)

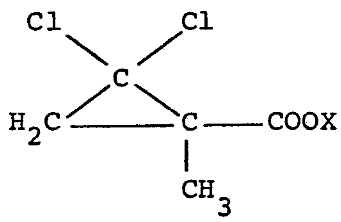

(II)

où R représente un atome d'hydrogène ou un groupe alcoyle comportant 1 à 6 atomes de C, en présence d'un catalyseur contenant un métal précieux connu, et en présence d'un accepteur de HCl, à une déchloruration hydrogénante sélective, l'acide libre étant préalablement transformé en sel, si X représente un atome d'hydrogène.

3. Procédé selon la revendication 2, caractérisé en ce que l'on effectue la déchloruration hydrogénante sélective en l'absence de solvant.

4. Procédé selon la revendication 2, caractérisé en ce que l'on effectue la déchloruration en présence d'eau.

5. Procédé selon l'une quelconque des revendications 2 à 4, caractérisé en ce que, comme accepteur d'HCl, on utilise des solutions aqueuses d'hydroxyde alcalin ou d'ammoniaque, ou de l'ammoniac anhydre ou des alcoolates.

6. Procédé selon l'une quelconque des revendications 2 à 5, caractérisé en ce que l'on opère sous une pression comprise entre 10 et 250 bars et à des températures comprises entre 20 et 120 °C.

7. Procédé selon l'une quelconque des revendications 2 à 6, caractérisé en ce que, comme catalyseur, on utilise du palladium ou du platine.

8. Procédé selon l'une quelconque des revendications 2 à 7, caractérisé en ce que, comme catalyseur, on utilise du palladium sur charbon actif.